# EUROPÄISCHE PATENTANMELDUNG

(11) **EP 4 176 730 A1**
(43) Veröffentlichungstag der Anmeldung: **10.05.2023**
(21) Anmeldenummer: 22186737.7
(22) Anmeldetag: 25.07.2022
(51) Int. Cl.: A23K 20/105, A23K 20/158, A23K 50/30, A61K 31/19, A61P 31/20, A01N 65/20, A01N 65/34, A23K 10/30, A23K 10/33, A23K 20/28, A23K 10/37, A61K 36/185, A61K 36/48, A61K 36/484, A61K 36/73, A61K 36/87, A61K 36/896, A61K 36/8962, A01N 37/04, A01N 37/36, A01N 65/08

(54) **ZUSAMMENSETZUNG ZUR REDUZIERUNG DER KONZENTRATION VON VIREN UND DES AFRIKANISCHEN SCHWEINEPEST-VIRUS (AFRICAN SWINE FEVER VIRUS, ASFV) IN TIERFUTTER UND EINSTREUMATERIAL FÜR STÄLLE**

(30) Priorität: 07.10.2021 EP 21201435
(71) Anmelder: Dr. Eckel Vermögensverwaltung GmbH, 56651 Niederzissen (DE)
(72) Erfinder: ECKEL, Bernhard, 56651 Niederzissen (DE); ECKEL, Viktor, 53474 Bad Neuenahr Ahrweiler (DE)
(74) Vertreter: Kutzenberger Wolff & Partner

(57) **Zusammenfassung**

Die Erfindung betrifft eine Zusammensetzung zur Reduzierung der Konzentration von Viren und des afrikanischen Schweinepest-Virus (African Swine Fever Virus, ASFV) in Tierfutter und organischen Einstreumaterialien.

## Beschreibung

Die vorliegende Erfindung betrifft die Verwendung einer Zusammensetzung umfassend Blütenstände und/oder ein oder mehrere Extrakte weiblicher Blütenstände der Gattung Humulus und/oder ein oder mehrere Extrakte von Glycyrrhiza und/oder ein oder mehrere Pflanzenteile von Glycyrrhiza zur Reduzierung der Konzentration von Viren und insbesondere des afrikanischen Schweinepest-Virus (African Swine Fever Virus, ASFV) in Tierfutter sowie in Einstreumaterial ("Einstreu") für Ställe (wie z.B. Stroh und/oder Strohpellets). Die Zusammensetzung kann dabei zusätzlich weitere Komponenten aufweisen, insbesondere eine oder mehrere Säuren wie Ameisensäure, Essigsäure, Milchsäure, Propionsäure, Buttersäure, Fumarsäure, Äpfelsäure, Weinsäure und/oder Citronensäure und/oder deren Ammonium-, Natrium-, Kalium-, Calcium- und/oder Magnesiumsalze und/oder ein oder mehrere Komponenten ausgewählt aus der Gruppe bestehend aus Traubentrester, Traubenkernextrakt, Traubenkerne, Akazienexsudat, Quillaya saponaria, Kieselgur, Seifenrindeextrakt, Weinrebenextrakt, 1,2-Propandiol, Roselle, Sapindus sapinoria, Trägerstoff E551 und Fließhilfsmittel und insbesondere eine beliebige Kombination der vorstehenden Komponenten enthalten.

Viren sind nach Büttner (in Rolle/Mayer, 2006: Medizinische Mikrobiologie, Infektions- und Seuchenlehre; 8. Auflage, MVS Medizinverlage Stuttgart) bei allen Arten von Lebewesen als obligat zellgebundene Mikroorganismen und Krankheitserreger zu finden. Gift heißt im lateinischen Virus. Laut Büttner (2006) sind Viren nur in lebenden Zellen vermehrungsfähig. Bekannte Viren in der Schweineproduktion sind u.a. Porcine Epidenic Diarrhea Virus (PEDV), Porcine Reproductive and Respiratory Syndrome Virus (PRRSV), Seneca Valley A Virus (SVA) sowie ASFV.

Der ASFV gilt als der Erreger, welcher die afrikanische Schweinepest auslöst. Durch Reduzierung des ASFV in Tierfutter oder Einstreumaterial kann somit ein Faktor ausgeschlossen und/oder reduziert werden, der als Vektor für die Ausbreitung der afrikanischen Schweinepest (African Swine Fever, ASF) gilt. Die vorgestellte Erfindung leistet somit einen Beitrag zur Eindämmung des afrikanischen Schweinepest-Virus und verbessert so die Biosicherheit in der landwirtschaftlichen Produktion.

Der Ausbruch der ASF hat erhebliche Auswirkungen auf die weltweite Eiweißversorgung mit einem negativen Einfluss auf die Schweinefleischproduktion und dessen Verbrauch (Woonwong et al., 2020: The future of the pig industry after the introduction of African Swine Fever into Asia; Animal Frontiere; Vol.10, No.4).

Laut FAO (Food and Agriculture Organisation of the United Nations) ist die Afrikanische Schweinepest eine hochgradig ansteckende, generalisierte Erkrankung von Schweinen, die durch ein Iridovirus der Familie der Asfarviridae verursacht wird.

Diese Viren weisen je nach Stamm eine unterschiedliche Virulenz auf. Sie zeichnen sich auch durch eine sehr hohe Widerstandsfähigkeit gegen physikalische und/oder chemische Inaktivierung aus. Der Erreger kann über lange Zeit in Blut, Kot und Gewebe lebensfähig bleiben. Er kann sich auch mittels anderer Vektoren (u.a. Einstreumaterialien und leeren Transportfahrzeugen von Lebendviehtransporten, die aus den betroffenen Gebieten zurückkehren) verbreiten. Daher hängt die Bekämpfung der ASF von der Ausrottungspolitik und der strengen Durchsetzung der Hygienepläne ab.

ASF tritt am häufigsten in der akuten Form als hämorrhagisches Fieber auf. Der ASF-Virus selber ist ein doppelsträngiges DNA-Arbovirus. Zugleich gilt er als einziges Mitglied der Familie der Asfarviridae.

Subakute und chronische Formen der Krankheit sind beschrieben. Die Mortalität liegt meistens bei nahezu 100%. Schweine jeden Alters sind betroffen.

Schweine gehören zu den Paarhufern und hier zur Unterordnung der Schweineartigen (Suina). Folgende Gattungen werden dazu gezählt: Sus (Wildschweine), Babyrousa (Hirscheber), Phacochoerus (Warzenschweine) sowie Potamochoerus (Buschschweine und Flussschweine) [vgl. auch Systematische Zoologie, Volker Storch, Ulrich Welsch; Spektrum Akademischer Verlag, 21.10.2003 - S. 854].

Vorbeugend verweist die FAO darauf, dass landwirtschaftliche Betriebe in betroffenen Regionen Maßnahmen zur Erhöhung der biologischen Sicherheit verschärfen müssen, einschließlich der Kontrolle der Futtermittel.

Die EFSA (European Food Safety Authority) weist in einer aktuellen Untersuchung (vgl. Ability of different matrices to transmit African swine fever virus; EFSA Journal 2021;19(4):6558) daraufhin, dass es unterschiedliche Faktoren gibt, die die Übertragung von afrikanischer Schweinepest bedingen. Dies können Rohstoffe zur Futtermittelproduktion, aber auch Futtermittel selbst sein. Auch Niederwerder et al. (vgl. Niederwerder et al., 2020: Mitigating the risk of African swine fever virus in feed with anti-viral chemical additives, Transbound Emerg Dis. 2021;68: 477 - 486) verweisen auf die Rolle der Futtermittel in der Übertragung des ASFV und damit der ASF.

Futter ist die Grundlage der Tierhaltung - das gilt für die landwirtschaftliche Nutztierhaltung und die nachgelagerte Ernährungsindustrie gleichsam, ebenso für die Haltung von Heim- und Hobbytieren (Eckdaten der Futtermittelbranche - Deutscher Verband Tiernahrung e.V. (dvtiernahrung.de))

Erfindungsgemäß kann (Tier-)Futter aufgefasst werden als Einzelfuttermittel, Kombination von Einzelfuttermitteln, Vormischungen daraus und/oder anderen Wirkstoffen und Alleinfuttermittel(n).

Wie bereits oben beschrieben, ist Einstreumaterial auch ein Vektor zur Übertragung von Viren und im speziellen auch ASF. Erfindungsgemäß kann Einstreumaterial aufgefasst werden als Stroh, Strohpellets oder andere organische, staubarme Materialien (siehe: Richtlinie für die artgerechte Schweinehaltung: hier NEULAND-Richtlinien für artgerechte Schweinehaltung; Stand April 2018). Da Einstreumaterialien ähnlich wie Futtermittel produziert werden, beziehungsweise bei deren Produktion als Nebenprodukt anfallen, muss davon ausgegangen werden, dass Einstreumaterialien eine ähnliche Rolle bei der Übertragung der ASF zuzuschreiben ist. Dies gilt insbesondere, da diese unter gleichen Bedingungen gelagert werden und intensiv mit dem Tier in Berührung kommen. Erfindungsgemäß wird das Einstreumaterial vor dem Einstreuen entsprechend den Dosierungen für Futter behandelt.

(Tier-)Futter im Sinne der Erfindung lässt sich wie folgt definieren:

**Tabelle 1: Erfindungsgemäße Definition von (Tier-)Futtermittel**

| **Gruppe** | **Produkte** | **wichtige Beispiele** |
|---|---|---|
| Nebenprodukte tierischer Herkunft zur Verwendung in Futtermittel (FM) | hydrolysiertes Protein | |
| | sprühgetrocknetes Blut und Plasma von Tieren | Schweineplasma |
| | | Rinderblut |
| | Fisch sowie andere Meerestiere, deren Erzeugnisse und Nebenerzeugnisse | Fischmehl |
| | Milcherzeugnisse | Milchpulver, Milchzucker, Molke |
| | Andere | Fleischknochenmehl |
| Einzelfuttermittel und Futterausgangserzeugnisse | Getreide, deren Erzeugnisse und Nebenprodukte | Mais, Weizen, Roggen, Gerste Hafer, Reis |
| | Ölsaaten und Ölfrüchte, deren Erzeugnisse und Nebenprodukte | Soja, Raps |
| | Andere Saaten und Früchte, deren Erzeugnisse und Nebenprodukte | Eicheln, Kastanien, Apfel, Trauben |
| | Grün- und Rauhfutter | Heu, Stroh |
| | Knollen, Wurzeln, ihre Erzeugnisse und Nebenerzeugnisse | Kartoffeln, Rüben, rote Beete |
| | Leguminosen, deren Produkte und Nebenprodukte | Erbsen, Acker- Sojabohnen |
| | Andere | |
| Mischfutter | Mehlig (Alleinfuttermittel) | Organische oder anorganische Stoffe in Mischungen, auch mit Zusatzstoffen, die zur Fütterung von Schweinen in Form von Alleinfuttermitteln, Ergänzungsfuttermitteln oder Vormischungen bestimmt sind |
| | Pelletiert (Alleinfuttermittel) | |
| | Flüssig (Alleinfuttermittel) | |
| | Mineralstoffe und Spurenelemente | |
| | sonstige Futterzusatzstoffe (Konservierungsmittel, Probiotika, Prebiotika, Aminosäuren und Aromen) | |

In WO2022/112365A1 wird eine Methode beschrieben, die Haltezeit zu verkürzen, die erforderlich ist, um den Virusabbau im Futter zu gewährleisten. In Futtermitteln kann die viruzide Wirkung von Benzoesäure durch ätherische Öle verstärkt werden. Eine Ausführungsform der Erfindung betrifft ein Verfahren zur zumindest teilweisen Inaktivierung von ASFV in Schweinefutter, dadurch gekennzeichnet, dass Benzoesäure zusammen mit mindestens einem ätherischen Öl dem Schweinefutter zugesetzt wird. Die bevorzugten ätherischen Öle sind Thymol und Eugenol.

In der WO 2020/149892 A1 wird eine Methode beschrieben, wie man mit Hilfe des dort beschrieben Futterzusatzes auf Basis von Formaldehyd und Propionsäure ASF in Zellkulturtest und Futter abschwächen kann.

In einer weiteren Veröffentlichung (WO 2020/180877 A1) wird eine Methode dargestellt, wie das Risiko einer Infektion mit Porcine Epidenic Diarrhea Virus (PEDV), Porcine Reproductive and Respiratory Syndrome Virus (PRRSV) sowie dem Seneca Valley A Virus (SVA) reduziert werden kann.

Einige Futtermittelzusätze wurden ebenfalls in der Arbeit von Trudeau et al., 2016, gegen PEDV untersucht und ihre Wirksamkeit zur Verringerung der infektiösen Viruskonzentration.

Trudeau et al., 2016 (Trudeau et al., 2016; Comparison of Thermal and Non-Thermal Processing for Swine Feed and the Use of Selected Feed additives on Inactivation of PEDV, Published: June 24, 2016) beschreiben auch, dass zukünftige Untersuchungen erforderlich sind, um die Prävalenz und Maximaltiter von PEDV in kommerziellen Schweinefuttermitteln zu bestimmen, um die Behandlungsparameter (Zeit und Temperatur der thermischen Behandlung und Dosis der eBeam-Behandlung (Applications of ionizing radiation technology (ebeam)) und gewählter Futtermittelzusatz) zu bestimmen, die für die Inaktivierung von PEDV am wirksamsten sind. WO 2020/180877 A1 und Trudeau et al., 2016 ziehen allerdings keinen Bezug zu ASFV.

Das Patent EP 3 054 783 B1 beschreibt eine Zusammensetzung von mittelkettigen Fettsäuren und eine Futterergänzung mit dieser Zusammensetzung. Es wird offenbart, dass die Erfindung zur Verbesserung der Darmgesundheit und der Immunität von Tieren sowie zur Eliminierung von schädlichen Krankheitserregern dient. In einer Ausführungsform enthält die Zusammensetzung, wie sie dort beschrieben ist, zusätzliche Rohmaterialien (Zusatzstoffe) und/oder wachstumsfördernde Substanzen. Die Zusatzstoffe sind in einer bevorzugten Ausführungsform ausgewählt aus der Gruppe u.a. bestehend aus Aromen, Pflanzenextrakten und Carbonsäuren. In den Ansprüchen 5 und 6 wird offenbart, dass die Formulierung auch gegen Viren wirkt, speziell Noroviren und den Rotavirus. In den Ansprüchen werden die Aromen, Pflanzenextrakte und Carbonsäuren jedoch nicht weiter erwähnt.

Die Veröffentlichung CN110279033A betrifft ein Verfahren zur Herstellung eines Futtermittelzusatzstoffes aus Hopfenextrakt. Der Humulus lupulus-Extrakt-Zusatz wird aus den folgenden Komponenten in Gewichtsteilen hergestellt: 20-50 Teile Humulus lupulus-Extrakt, 20-30 Teile Süßholzpulver und 20-60 Teile Maiskolbenmehl. Außerdem wird offenbart, dass deren Formulierung den Gesundheitszustand und die Immunität des Organismus verbessern, sowie die Futterverwertung reduzieren. Des Weiteren wird auf die Bedeutung der Erfindung zur Antibiotikareduzierung verwiesen.

Die Veröffentlichung US2021/298311A1 beschreibt eine Zusammensetzung zur Inaktivierung von Schweineviren und entsprechende Verfahren im Futter. Ätherische Öle im Sinne der Patentansprüche entstammen den Ölen von Oregano, Capsicum, Rosemarin, Zimt, Thymian, Zitronengras, Bergamotte und Kurkuma. Diese Erfindung schließt explizit Substanzen aus der Gruppe der Fettsäuren, Formaldehyde und organischen Säuren in der Kombination aus.

Die Veröffentlichung CN110664914A beschreibt ein Verfahren, basierend insbesondere auf Fermentation entsprechend Chinesischer Medizin, um den ASFV zu reduzieren. Explizit werden die zu fermentierenden Pflanzen (Substrat), sowie die Mikroorganismen (Hefe, Milchsäurebakterien sowie Bacillus subtilis) beschrieben, die prozessrelevant sind. Aufgrund der Fermentation und dem beschriebenen Vorgehen und Fokus auf Fermentation ist jedoch davon auszugehen, dass die beschriebene Wirkung aufgrund der Fermentation des Substrats selbst beruht.

In CN110651903A wird eine Methode beschrieben, ein Schweinefutter basierend auf Wermut, zur Reduzierung des ASFV. Hierbei werden explizit neben Kräutern, Vitaminen, Enzymen Säuren nur zur Ansäuerung eingesetzt.

CN111213786A und CN109497107A beschreiben Methoden durch Trinkwasserapplikation sowie eine Methode zur Desinfektion von luftübertragbaren Krankheiten wie ASF. Beide Verfahren sind demnach auf Futter und Einstreumaterialien nicht anwendbar.

Über eine Inaktivierung von ASF-Viren in Einstreumaterial für Ställe ist bisher wenig bekannt.

Die aus dem Stand der Technik bekannten Zusammensetzungen sind nicht in jeder Hinsicht zufriedenstellend.

Eine Aufgabe der vorliegenden Erfindung ist es daher, Zusammensetzungen bzw. Kombinationen zur Verfügung zu stellen, die eine Reduzierung der Konzentration von Viren, insbesondere des ASFV in Tierfutter, bevorzugt im Futter für Schweine bewirkt.

Die vorstehend genannte Aufgabe wurde durch den Gegenstand der Patentansprüche gelöst.

Des Weiteren hilft die vorliegende Erfindung auch die Virenlast in Einstreumaterial insbesondere basierend auf Stroh, Strohpellets oder anderen organischen, staubarmen Materialien nachhaltig zu reduzieren.

Die Erfinder konnten zeigen, dass die Menge von Viren, insbesondere ASFV, in Tierfutter signifikant reduziert wurde, insbesondere durch erfindungsgemäße Verwendung.
Figur 1 zeigt die Bestimmung der ASFV-Kinetik nach Virusinokulation des Futtersmittels rtPCR (Ct Werte > 40 negativ; < 40 positiv).
Figur 2 zeigt die Bestimmung der ASFV-Kinetik nach Virusinokulation mittels rtPCR (Ct Werte > 40 negativ; < 40 positiv).
Figur 3 zeigt die Bestimmung der ASFV-Kinetik nach der Virusinokulation mittels HAD50.
Figur 4 zeigt die Bestimmung der ASFV-Kinetik nach der Virusinokulation mittels HAD50.
Figur 5 zeigt, dass die erfindungsgemäßen Substanzen entweder additiv oder multiplikativ eine verstärkte Wirkung erreichen.

Die Erfindung betrifft die Verwendung einer Zusammensetzung umfassend
(i) Blütenstände und/oder ein oder mehrere Extrakte weiblicher Blütenstände der Gattung Humulus und/oder
(ii) ein oder mehrere Extrakte von Glycyrrhiza und/oder ein oder mehrere Pflanzenteile von Glycyrrhiza, vorzugsweise von Wurzeln, Wurzelausläufern und/oder Wurzelstock,
zur Reduzierung
- von Viren (bzw. deren Konzentration) in Einstreumaterialien, und/oder
- von Viren (bzw. deren Konzentration) in Futter, vorzugsweise Tierfutter, vorzugsweise
- des ASF-Virus (bzw. dessen Konzentration) in Einstreumaterialien, und/oder
- des ASF-Virus (bzw. dessen Konzentration) in Futter, vorzugsweise Tierfutter.

Dabei beziehen sich im Sinne der Erfindung "Extrakte weiblicher Blütenstände der Gattung Humulus" insbesondere auf die Gesamtweichharze des Hopfens, isomerisiert oder nicht. Dazu gehören u.a. Alphasäureextrakte des Hopfens sowie deren isomerisierte Iso-alpha-Säuren (zu den Alphasäuren und deren Isomeren gehören u.a. Humulon, Cohumulon sowie Adhumolon und Isohumulon, Isocohumulon sowie Isoadhumulon), möglichst frei von Betasäuren und Hopfenölen. Außerdem zählen zu den Hopfenextrakten Betasäureextrakte (möglichst frei von Alphasäuren und Hopfenölen) des Hopfens sowie deren isomerisierten Iso-beta-Säuren. Zu den β-Säuren gehören u.a. Lupulon, Colupulon und Adlupulon.

Des Weiteren umfassen synthetische Alpha- und Betasäuren bzw. deren isomerisierte Formen sowie Hopfendoldenpellets, Hopfendoldenpulver sowie der Extraktionstreber des Hopfens vorzugsweise die erfindungsgemäße Komponente (i).

Ferner bezieht sich im Sinne der Erfindung "Extrakte von Glycyrrhiza" insbesondere auf die Verwendung von trockenem, wasserlöslichen und/oder flüssigem Süßholzextrakt und/oder Süßholzpulver.

### Vorzugsweise

- beträgt die Gesamtmenge der (i) Blütenstände und/oder Extrakte weiblicher Blütenstände der Gattung Humulus und/oder (ii) Extrakte von Glycyrrhiza und/oder Pflanzenteile von Glycyrrhiza mindestens 20 Gew.-% der Zusammensetzung, bezogen auf das Gesamtgewicht der Zusammensetzung; und/oder
- ist das Verhältnis, vorzugsweise das Gewichtsverhältnis, der (i) Blütenstände und/oder Extrakte weiblicher Blütenstände der Gattung Humulus und/oder (ii) Extrakten von Glycyrrhiza und/oder Pflanzenteilen von Glycyrrhiza zwischen 0,5 und 3,5, besonders bevorzugt zwischen 0,6 und 3,3 und ganz besonders bevorzugt zwischen 0,7 und 3,0; und/oder
- ist die Dosierung im Futter oder der organischen Einstreu, bezogen auf die Gesamtmenge der Trockensubstanz des Futters, 0,01 Gew-% bis 0,15 Gew-% und bevorzugt 0,03 Gew-% bis 0,12 Gew-%, und besonders bevorzugt 0,04 Gew-% bis 0,10 Gew-%; und/oder
- enthält die Zusammensetzung zusätzlich
- (iii) ein oder mehrere Komponenten unabhängig voneinander ausgewählt aus der Gruppe bestehend aus Traubentrester, Traubenkernextrakt, Traubenkerne, Akazienexsudat, Quillaya saponaria, Seifenrindeextrakt, Weinrebenextrakt, Roselle, Sapindus sapinoria, Kieselgur, E551 (a-c) und 1,2-Propandiol, und/oder,
- (iv) ein oder mehrere Komponenten ausgewählt aus der Gruppe bestehend aus Ameisensäure, Essigsäure, Milchsäure, Propionsäure, Buttersäure, Fumarsäure, Äpfelsäure, Weinsäure und Citronensäure, wobei die Säuren teilweise oder vollständig in Form von deren Ammonium-, Natrium-, Kalium-, Calcium- und/oder Magnesiumsalzen vorliegen können;
- und/oder Kombinationen aus (iii) und (iv) davon; jeweils unabhängig voneinander, entweder einzeln oder in Kombination, vorzugsweise in einer Menge von mindestens 1 Gew.-%, mindestens 5 Gew.-%, mindestens 10 Gew.-%, mindestens 20 Gew.-%, mindestens 30 Gew.-%, mindestens 40 Gew.-% oder mindestens 50 Gew.-%, jeweils bezogen auf das Gesamtgewicht der Zusammensetzung; und/oder
- erfolgt die Gesamtdosierung der Zusammensetzung aus (i), (ii), (iii) und/oder (iv) zwischen 0,1 Gew.-% bis 1,5 Gew.-%, bevorzugt 0,3 Gew.-% bis 1,0 % Gew.-% und besonders bevorzugt 0,4 Gew.-% bis 0,8 Gew.-%, bezogen auf das Gesamtgewicht der Trockensubstanz des Futters oder der organischen Einstreu; und/oder
- enthält die Zusammensetzung zwischen 1,5 Gew.-% und 12 Gew.-%, bevorzugt zwischen 2 Gew.-% und 11 Gew.-% oder zwischen 3 Gew.-% und 10 Gew.-%, der Komponenten (i) und (ii), bezogen auf das Gesamtgewicht der Zusammensetzung.

Im Sinne der Erfindung können die Begriffe "Einstreumaterial" und "Einstreu" synonym verwendet werden.

Die Erfindung betrifft in einem weiteren Aspekt eine entsprechende Zusammensetzung als solche.

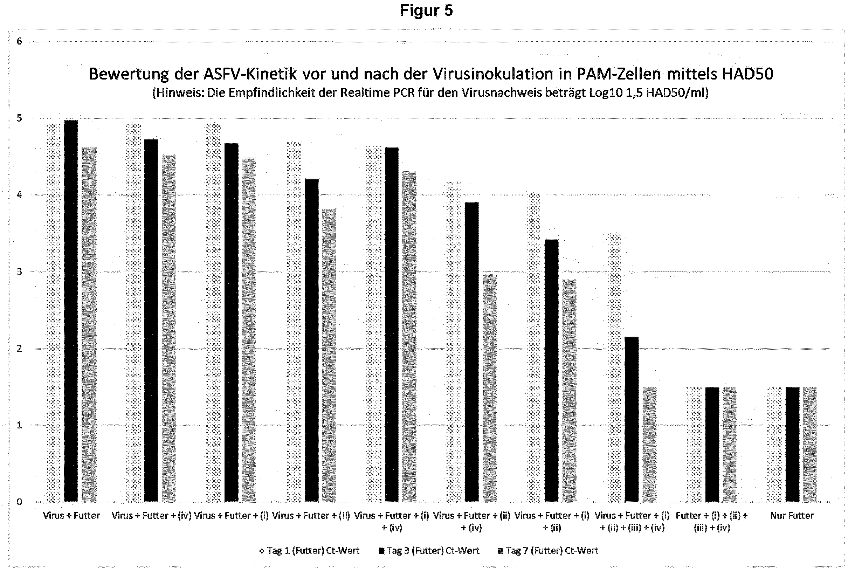

Die erfindungsgemäßen Komponenten sind jeweils einzeln oder in Kombination miteinander einzusetzen. Dabei erreichen diese Substanzen entweder additiv oder multiplikativ eine verstärkte Wirkung (siehe auch Figur 5). Die Komponenten, insbesondere die Extrakte, können flüssig oder fest vorliegen und dabei jeweils entweder Reste des Extraktionsmittels enthalten oder nicht. Erfindungsgemäß können diese Extrakte entweder flüssig oder fest vorliegen sowie natürlich, naturidentisch oder synthetisch sein.

Für flüssige Formulierungen der Erfindung wird als Träger bevorzugt 1,2-Propandiol (Propylenglycol) benutzt.

Die Applikation in den Einzelfuttermitteln, Vormischungen und Alleinfuttermitteln erfolgt durch Einmischen ins Tierfutter und Einstreumaterialien bzw. durch das Sprühen der erfindungsgemäßen flüssigen Bestandteile während des Mischvorgangs in das Tierfutter und Einstreumaterialien bzw. durch eine Applikation nach dem Mischen und/oder Pelletieren und/oder Granulieren. Die erfindungsgemäße Applikation erfolgt bevorzugt bei einem erhöhten ASFV-Risiko und besonders bevorzugt kontinuierlich.

In einer bevorzugten Ausführungsform werden die Komponenten (i) und/oder (ii) jeweils allein oder in Kombination einzeln und besonders bevorzugt in einem Kombinationspräparat mit Komponenten (iii) und/oder (iv) den Einzelfuttermitteln, Vormischungen und Alleinfuttermitteln und Einstreumaterialien verabreicht.

In einer anderen bevorzugten Ausführungsform werden die Komponenten (i) und/oder (ii) und/oder (iii) und/oder (iv) in Form einer Vormischung, vorzugsweise in Form einer vorgemischten Zusammensetzung jeweils Komponente (i) oder (ii) oder (iii) oder (iv) alleine, bevorzugt wenigstens zwei der Komponenten (i) und/oder (ii) und/oder (iii) und/oder (iv besonders vorzugsweise die Komponenten (i) und (ii) verabreicht, vorzugsweise indem sie den Einzelfuttermitteln, Vormischungen und Alleinfuttermitteln und Einstreumaterialien beigemischt werden. Erfindungsgemäß können die Komponenten (i), (ii), (iii) oder (iv) jeweils alleine verabreicht werden. Besonders bevorzugt werden die Komponenten (i), (ii), (iii) und/oder (iv)jedoch gemeinsam verabreicht. Die erfindungsgemäße Zusammensetzung aus den Komponenten (i) und/oder (ii) und/oder (iii) und/oder (iv) kann jeweils einzeln und/oder mindestens einer Vormischung sowohl flüssig und/oder fest verabreicht werden.

Vorzugsweise enthält die Zusammensetzung kein Maiskolbenmehl.

Die nachfolgenden Beispiele dienen der Erläuterung der Erfindung, schränken aber den allgemeinen Erfindungsgedanken nicht ein.

### Beispiele:

Als Beispiele für die erfindungsgemäße Zusammensetzung sind nachfolgend exemplarisch einige typische Zusammensetzungen einer Tierfutterzusatzmischung aufgeführt, wobei die Angaben jeweils in Gewichtsprozent bezogen auf das Gesamtgewicht der Zusammensetzung erfolgen.

### Beispiel 1:

| | |
|---|---|
| Hopfenextraktionstreber | 50% |
| Hopfenextrakt | 20% |
| Traubentrester | 16% |
| Süßholz | 4% |
| Akazienexsudat | 4% |
| Quillaya saponaria | 2% |
| Fliesshilfsmittel | 4% |

### Beispiel 2:

| | |
|---|---|
| Hopfenextraktionstreber | 50% |
| Traubentrester | 25% |
| Hopfenextrakt | 15% |
| Süßholz | 5% |
| Fliesshilfsmittel | 5% |

### Beispiel 3:

| | |
|---|---|
| Kieselgur | 40% |
| Milchsäure | 30% |
| Calciumformiat | 20% |
| Citronensäure | 5% |
| Fliesshilfsmittel | 5% |

### Beispiel 4:

| | |
|---|---|
| Hopfentreber | 10% |
| Hopfenextrakt | 10% |
| Traubentrester | 5% |
| Süßholzpulver | 4% |
| Calciumpropionat | 36% |
| Fumarsäure | 25% |
| Calciumlactat | 10% |

### Beispiel 5:

| | |
|---|---|
| Äpfelsäure | 20% |
| Natriumformiat | 20% |
| Magnesiumfumarat | 15% |
| Natriumacetat | 10% |
| Citronensäure | 10% |
| Fumarsäure | 5% |
| Seifenrindeextrakt | 9% |
| Hopfenextrakt | 6% |
| Weinrebenextrakt | 5% |

### Beispiel 6:

| | |
|---|---|
| Calciumformiat | 80% |
| Seifenrindenextrakt | 11% |
| Milchsäure | 5% |
| Hopfen | 4% |

### Beispiel 7:

| | |
|---|---|
| Calciumlactat | 30% |
| Calciumformiat | 20% |
| Milchsäure | 10% |
| Hopfen | 10% |
| Süßholzextrakt | 5% |
| Quillaya saponaria | 5% |
| Traubentreber | 5% |
| Trägerstoff E551 | 15 % |

### Beispiel 8:

| | |
|---|---|
| Calciumformiat | 50% |
| Calciumlactat | 30% |
| Citronensäure | 12% |
| Hopfen | 5% |
| Traubenkernextrakt | 2% |
| Süßholz | 1% |

### Beispiel 9:

| | |
|---|---|
| Traubentrester | 2% |
| Traubenkerne | 2% |
| Roselle | 2% |
| Hopfendolden | 10% |
| Seifenrindenextrakt | 4% |
| Sapindus sapinoria | 2% |
| Ameisensäure | 30% |
| Magnesiumfumarat | 16% |
| Citronensäure | 10% |
| Träger E551 | 20% |

### Beispiel 10:

| | |
|---|---|
| Hopfenextrakt | 0,4% |
| Calciumformiat | 70,6% |
| Calciumlactat | 12% |
| Citronensäure | 17% |

### Beispiel 11:

| | |
|---|---|
| Hopfenextrakt | 0,5% |
| Hopfentreber | 4% |
| Calciumformiat | 68% |
| Calciumlactat | 9,5% |
| Citronensäure | 15% |
| Anorganischer Träger (E551) | 3% |

### Beispiel 12:

| | |
|---|---|
| Hopfenextrakt | 7% |
| Süssholzextrakt | 8% |
| Calciumsalz der Ameisensäure | 25 % |
| Citronensäure | 10 % |
| Hopfentreber | 50 % |

### Beispiel 13:

| | |
|---|---|
| Propionsäure | 10% |
| 1,2-Propandiol | 77% |
| Süssholz | 8% |
| Hopfenextrakt | 5% |

### Beispiel 14:

| | |
|---|---|
| Hopfenextrakt | 0,4% |
| Hopfentreber | 3,4 % |
| Süßholz | 0,6% |
| Calciumformiat | 69,1% |
| Calciumlactat | 11% |
| Citronensäure | 14% |
| E551 | 1,5% |

Um die Eigenschaften der erfindungsgemäß verwendeten Kombination zu überprüfen, wurde ein kommerzielles, praxisübliches Schweinefutter beschafft (Umsetzbare Energie: > 3150 kcal/kg, Rohprotein> 17,5 %, Rohfaser ≤ 5,5 %, Calcium 0,7 - 1,4 %; Phosphor total 0,5 - 1,0 %; gesamt Lysin ≥ 1,0 %, Methionin + Cystein ≥ 0,6 %, Trockensubstanz ≥ 87 %).

Dieses Schweinefutter war frei von Antibiotika und Formaldehyd. Komponenten waren u.a. Getreide, Soja, Nebenprodukte von Getreide, Fleischknochenmehl, Vitamine und Mineralstoffe.

Als ASFV wurde der Stamm VNUA/HY-ASF1/Vietnam/2019 zum Beimpfen des Futters benutzt. Das Virusisolat wurde der Sammlung des Department of Microbiology and Infectious Disease, Key Laboratory for Veterinary Biotechnology, Vietnam National University of Agriculture (VNUA) entnommen. Er gehört zum p72 genotype II, der zu 100% identisch ist mit dem hochpathogenen ASFV Stamm, der in China (2018) und Georgien (2007) isoliert wurde. Die Impfdichte des ASFV war 10⁸ HAD₅₀/ ml.

Zur Untersuchung der Wirkung der erfindungsgemäßen Kombination auf das ASFV im Futter wurden zwei Methoden verwendet. Zum einen wurde ein rtPCR-(Echtzeit Polymerase Kettenreaktion)-Nachweis durchgeführt. Dazu wurde ein kommerzieller rtPCR-Test auf ASFV der Firma Median Diagnostics Inc. (http://www.mediandiagnostics.com) verwendet. Dieser Test weist die Präsenz der viralen DNA in der Probe nach. Zusätzlich wurde die Aktivität der enthaltenen Viren mittels Haemadsorptionstest nachgewiesen. Bei diesem Test werden aktive Viren durch Haemadsorptionsreaktion mit Schweineblut nachgewiesen.

Dem Fachmann sind beide Methoden hinreichend bekannt, sie sind als Methoden zur ASFV-Analytik etabliert.

Das Vorgehen für den rtPCR-Test erfolgte entsprechend der Herstellerangaben.

Das Vorgehen für den Haemadsorptionstest war wie folgt: Porcine Makrophagen (entweder BMDM, Bone Marrow-Derived Macrophages oder PAM, Porcine alveolare makrophagen) wurden einen Tag vor dem Experiment aus Schweinen mittels RPMI-Medium gewonnen. Die BMDM/PAM-Zellen wurden gesammelt, gewaschen und auf 96-Well-Platten mit 100µl Zelle/Well kultiviert. Die Freiheit des Schweinefutters, der Komponenten und der Reagenzien vom ASFV wurde mittels rtPCR sichergestellt. Jeweils 100 g Schweinefutter wurden aliquotiert. Die Proben wurden dann mit den erfindungsgemäßen Komponenten behandelt. Dann wurden zur Viruskontamination 1 ml ASFV (VNUA/HY-ASF1/Vietnam/2019)-Lösung (10⁸ HAD₅₀/ml) zu jeder Probe zugefügt. Die Proben wurden zur Homogenisierung 10 Minuten lang geschüttelt. Alle Proben wurden bei Raumtemperatur (25 °C) inkubiert. Nach einer Inkubation von 1, 3 und 7 Tagen wurde das Überleben des Virus bewertet. Hierbei wurden jeweils die folgenden Gruppen untersucht:
Futter und Virus (Positivkontrolle)
Futter und Virus und Komponente (i), (ii) und (iv)
Futter und Virus und Komponente (iv)
Futter und Virus und Komponenten (i), (ii), (iii) und (iv)
Futter (Negativkontrolle 1)
Futter und Komponenten (i), (ii), (iii) und (iv) (Negativkontrolle 2)

Es wurden die Rezepturen (bzw. deren Komponenten) eingesetzt, jeweils bezogen auf 5400 g / to Applikation im Futter. Als Komponenten kamen Extrakte weiblicher Blütenstände der Gattung Humulus insbesondere Gesamtweichharze und Hopfenextraktionstreber, (ii) Extrakte von Glycyrrhiza (iii) E551 sowie (iv) Citronenensäure und die Calciumsalze der Ameisen- und Milchsäure im Verhältnis 15:70:13 zum Einsatz. Alle Komponenten waren, zur Zeit der Versuchsdurchführung, in der europäischen Futtermittelgesetzgebung gelistet, Stand 2021.

Nach der Inkubationszeit wurden 50 ml RPMI-Medium in jeden Beutel gegeben. Nach dem Mischen wurde der Überstand aufgefangen. Der resultierende Überstand wurde durch einen 0,22-µm-Filter gefiltert, um Verunreinigungen und Bakterien zu entfernen. Die Proben wurden 10-fach in RPMI-Medium verdünnt. In jede Vertiefung der vorkultivierten PAM/BMDM-Zellplatten wurden 100 µl der verdünnten Probe gegeben, jede Probenverdünnung wurde in 3 Vertiefungen gegeben. Die Platten wurden bei 37 °C und 5 % CO₂ 2 Stunden lang inkubiert, dann wurde die Mischung entfernt und einmal mit 1X PBS gewaschen. Nach Zugabe von 200µL Erhaltungsmedium (10 % FBS + RPMI + 1 % Antibiotikum) zu den Platten folgte eine erneute Inkubation bei 37 °C und 5 % CO₂. für 48 Stunden. Dann wurden jeder Vertiefung 20 µl 0,1 % rote Blutkörperchen vom Schwein (pRBC) in RPMI-Medium zugesetzt. Danach wurde der zytopathischen Effekts (CPE) und der Zelllyse täglich bestimmt. Die Virustiter wurden als HAD₅₀ berechnet. Außerdem wurde die Menge (Ct-Werte) von ASFV mittels kommerzieller rtPCR (Median Diagnostics Inc., http://www.mediandiagnostics.com) bestimmt.

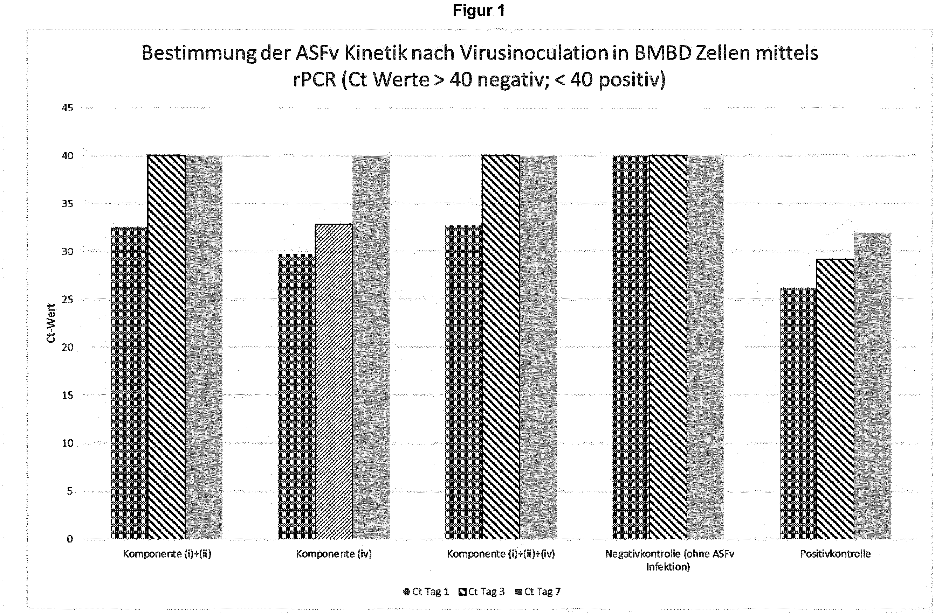

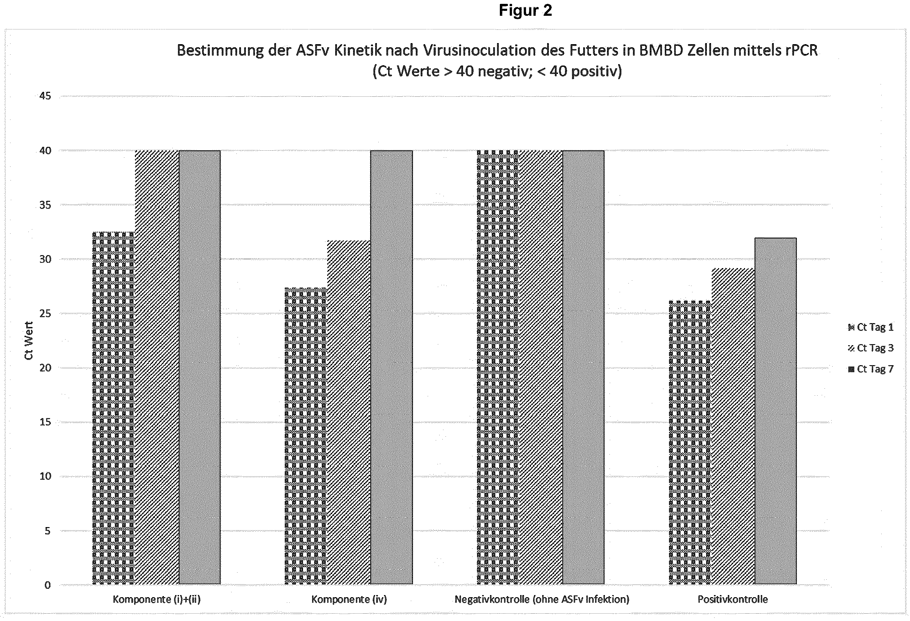

Die Ergebnisse der rtPCR zeigten, dass Komponente (i)+(ii), die Komponente (iv) und die Kombination der Komponenten (i), (ii) und (iv) in unterschiedlichen Konzentrationen das genetische Material von ASPV im Futter an Tag 1 mit höheren Ct-Werten im Vergleich zur Positivkontrolle reduzierten. Die Beobachtung verstärkte sich am Tag 3. Am 7. Tag war das genetische Material von ASFV im Futter im Vergleich zur Positivkontrolle deutlich reduziert (Figuren 1 und 2).

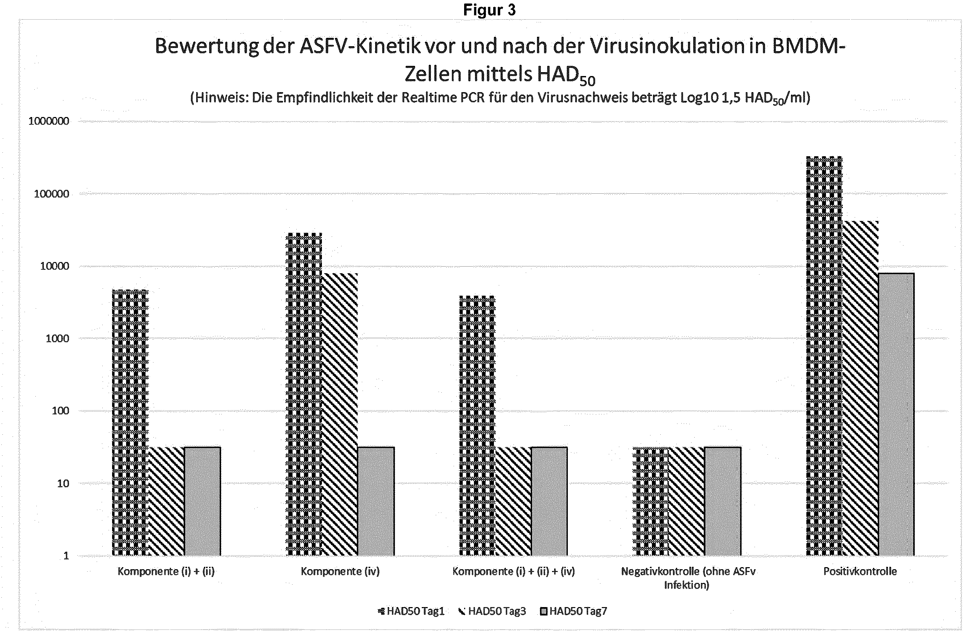

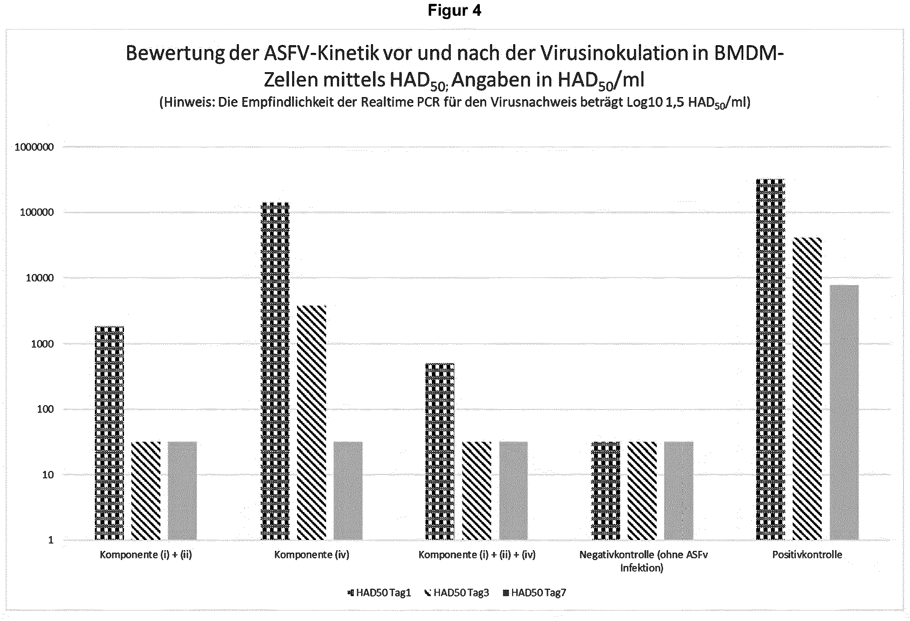

In Übereinstimmung mit den rtPCR-Ergebnissen zeigte der Haemadsorptionstest, dass Komponente B die Aktivität des ASFV im Futter von Tag 1 bis 7 signifikant reduzierte (Figuren 3 und 4).

Die Behandlungen mit Komponente (i) +(ii) und Komponente (iv) ergaben ein eindeutiges Ergebnis.

Am Tag 1 war ein deutlicher Rückgang der viralen HADso-Werte bei verschiedenen Konzentrationen zu verzeichnen (Figuren 3 und 4).

Insbesondere an Tag 7 zeigten die Ergebnisse eine vollständige Unterdrückung des Virus.

Die Kombination (i), (ii) und (iv) ergaben sehr positive Ergebnisse an Tag 1 und Tag 3, an Tag 7. Eine deutliche virale Unterdrückung wurde gemessen.

Die Ergebnisse der Versuche zeigten, dass die Komponenten (i), (ii) und (iv) eine starke synergistische Wirkung zur Verringerung der Überlebensfähigkeit von ASPV in Futtermitteln hatten und eine schnelle Verringerung der Menge an ASPV-DNA im untersuchten Futter sowie der Menge an infektiösem ASPV im untersuchten Futter haben. Nach 7 Tagen waren die jeweils erreichten Werte im Bereich der Negativkontrolle, der kein Virus zugefügt wurde. Dieser Versuch lässt auch den Schluss zu, dass Komponenten (i) + (ii) und Komponente (iv) jeweils alleine oder bevorzugt in einer Kombination der Komponenten (i) und (ii) + (iv) das Risiko einer ASPV-Übertragung durch Futtermittel verringern können und somit einen sehr starken Beitrag zur Biosicherheit von Futtermitteln leisten. Der Einsatz der Komponenten (i), (ii), (iii) und (iv) jeweils allein oder in Kombination dient somit prophylaktisch der Reduzierung des ASFV im Futter und Einstreumaterialien.

In einem weiteren Versuch (F 5) wurden die Komponenten (i), (ii), (iii) und (iv) einzeln und in der Kombination getestet. Auch hier zeigte sich überraschender Weise die Vorzüglichkeit der Erfindung. Nach 7 Tagen war kein Virus nachweisbar.

Weitere bevorzugte Ausführungsformen ("AF") der vorliegenden Erfindung sind die folgenden Ausführungsformen AF1-AF20. Unter der Reduzierung der Konzentration von Viren und/oder des afrikanischen Schweinepest-Virus (ASFV) wird hierbei insbesondere die Reduzierung der Konzentration von einem Virus oder mehreren Viren ausgewählt aus der Gruppe bestehend aus Porcine Epidenic Diarrhea Virus (PEDV), Porcine Reproductive and Respiratory Syndrome Virus (PRRSV), Seneca Valley A Virus (SVA) und ASFV verstanden, vorzugsweise des afrikanischen Schweinepest-Virus (ASFV) verstanden.
- AF1:: Verwendung eines Alphasäureextrakts oder synthetischer Alphasäure des Hopfens sowie deren isomerisierten Iso-alpha-Säuren (zu den Alphasäuren und deren Isomeren gehören u.a. Humulon, Cohumulon sowie Adhumolon und Isohumulon, Isocohumulon sowie Isoadhumulon), möglichst frei von Betasäuren und Hopfenölen zur (i) Reduzierung der Konzentration von Viren und/oder des afrikanischen Schweinepest-Virus (ASFV) in Tierfutter sowie organischen Einstreumaterial, oder (ii) Inaktivierung von Viren und/oder des afrikanischen Schweinepest-Virus (ASFV) in Tierfutter sowie organischen Einstreumaterial.
- AF2:: Verwendung eines Betasäureextrakts oder synthetischer Betasäure (möglichst frei von Alphasäuren und Hopfenölen) des Hopfens zur (i) Reduzierung der Konzentration von Viren und/oder des afrikanischen Schweinepest-Virus (ASFV) in Tierfutter sowie organischen Einstreumaterials, oder (ii) Inaktivierung von Viren und/oder des afrikanischen Schweinepest-Virus (ASFV) in Tierfutter sowie organischen Einstreumaterial. Zu den ß-Säuren gehören u.a. Lupulon, Colupulon und Adlupulon.
- AF3:: Verwendung eines Aromaextrakts des Hopfens (möglichst frei von Hopfensäuren) zur (i) Reduzierung der Konzentration von Viren und/oder des afrikanischen Schweinepest-Virus (ASFV) in Tierfutter sowie organischen Einstreumaterial, oder (ii) Inaktivierung von Viren und/oder des afrikanischen Schweinepest-Virus (ASFV) in Tierfutter sowie organischen Einstreumaterial.
- AF4:: Verwendung anderer Extrakte des Hopfens zur (i) Reduzierung der Konzentration von Viren und/oder Reduzierung der Konzentration des afrikanischen Schweinepest-Virus (ASFV) in Tierfutter sowie organischen Einstreumaterial oder (ii) Inaktivierung von Viren und/oder des afrikanischen Schweinepest-Virus (ASFV) in Tierfutter sowie organischen Einstreumaterial.
- AF5:: Verwendung des Extraktionstrebers des Hopfens zur (i) Reduzierung der Konzentration von Viren und/oder des afrikanischen Schweinepest-Virus (ASFV) in Tierfutter sowie organischen Einstreumaterial, oder (ii) Inaktivierung von Viren und/oder des afrikanischen Schweinepest-Virus (ASFV) in Tierfutter sowie organischen Einstreumaterial.
- AF6:: Verwendung von Hopfendoldenpulver zur (i) Reduzierung der Konzentration des von Viren und/oder des afrikanischen Schweinepest-Virus (ASFV) in Tierfutter sowie organischen Einstreumaterial, oder (ii) Inaktivierung von Viren und/oder des afrikanischen Schweinepest-Virus (ASFV) in Tierfutter sowie organischen Einstreumaterial.
- AF7:: Verwendung einer Kombination aus AF 1-6 zur (i) Reduzierung der Konzentration von Viren und/oder des afrikanischen Schweinepest-Virus (ASFV) in Tierfutter sowie organischen Einstreumaterial, oder (ii) Inaktivierung von Viren und/oder afrikanischen Schweinepest-Virus (ASFV) in Tierfutter sowie organischen Einstreumaterial.
- AF8:: Verwendung von trockenem oder flüssigem Süßholzextrakt zur (i) Reduzierung der Konzentration von Viren und/oder des afrikanischen Schweinepest-Virus (ASFV) in Tierfutter sowie organischen Einstreumaterial, oder (ii) Inaktivierung von Viren und/oder des afrikanischen Schweinepest-Virus (ASFV) in Tierfutter sowie organischen Einstreumaterial.
- AF9:: Verwendung von Süßholzpulver zur (i) Reduzierung der Konzentration von Viren und/des afrikanischen Schweinepest-Virus (ASFV) in Tierfutter sowie organischen Einstreumaterial., oder (ii) Inaktivierung von Viren und/oder des afrikanischen Schweinepest-Virus (ASFV) in Tierfutter sowie organischen Einstreumaterial.
- AF10:: Verwendung einer Kombination von AF 8 und 9 zur (i) Reduzierung der Konzentration von Viren und/oder des afrikanischen Schweinepest-Virus (ASFV) in Tierfutter sowie organischen Einstreumaterial, oder (ii) Inaktivierung von Viren und/oder des afrikanischen Schweinepest-Virus (ASFV) in Tierfutter sowie organische Einstreumaterial.
- AF11:: Verwendung einer Kombination aus einem der AF 1-7 und einem der AF 8-10 zur (i) Reduzierung der Konzentration von Viren und/oder des afrikanischen Schweinepest-Virus (ASFV) in Tierfutter sowie organischen Einstreumaterial, oder (ii) Inaktivierung von Viren und/oder des afrikanischen Schweinepest-Virus (ASFV) in Tierfutter sowie organischen Einstreumaterial.
- AF12:: Verwendung von einem oder mehreren Extrakten und/oder Bestandteilen wenigstens einer Pflanze ausgewählt aus der Gruppe (iii) bestehend aus Traubentrester, Traubenkernextrakt, Traubenkerne, Akazienexsudat, *Quillaya saponaria,* Seifenrindeextrakt, Weinrebenextrakt, Roselle, *Sapindus sapinoria,* Kieselgur, E551 (a-c) und 1,2-Propandiol, zur (i) Reduzierung der Konzentration von Viren und/oder des afrikanischen Schweinepest-Virus (ASFV) in Tierfutter sowie organischen Einstreumaterial, oder (ii) Inaktivierung von Viren und/oder des afrikanischen Schweinepest-Virus (ASFV) in Tierfutter sowie organischen Einstreumaterial.
- AF13:: Verwendung von einer oder mehreren der folgenden Säuren und deren Salzen: (iv) Ameisensäure, Essigsäure, Milchsäure, Propionsäure, Buttersäure, Fumarsäure, Äpfelsäure, Weinsäure und/oder Citronensäure; und/oder deren Ammonium-, Natrium-, Kalium-, Calcium- und/oder Magnesiumsalze , zur (i) Reduzierung der Konzentration von Viren und/oder des afrikanischen Schweinepest-Virus (ASFV) in Tierfutter sowie organischen Einstreumaterial, oder (ii) Inaktivierung von Viren und/oder des afrikanischen Schweinepest-Virus (ASFV)
- ;: in Tierfutter sowie organischen Einstreumaterial.
- AF14:: Verwendung einer Kombination aus einem der AF 1-7 und/oder einem der AF 8-13 und/oder dem AF 12 zur (i) Reduzierung der Konzentration von Viren und/oder des afrikanischen Schweinepest-Virus (ASFV) in Tierfutter sowie organischen Einstreumaterial, oder (ii) Inaktivierung von Viren und/oder des afrikanischen Schweinepest-Virus (ASFV) in Tierfutter sowie organischen Einstreumaterial.
- AF15:: Verwendung einer Kombination nach AF 14 zur (i) Reduzierung der Konzentration von Viren und/oder des afrikanischen Schweinepest-Virus (ASFV) in Tierfutter sowie organischen Einstreumaterial, oder (ii) Inaktivierung von Viren und/oder des afrikanischen Schweinepest-Virus (ASFV) in Tierfutter sowie organischen Einstreumaterial.
- AF16:: Verwendung einer Kombination aus einem der AF 1-7 und/oder einem der AF 8-10 und/oder dem AF12-13 und/oder einem der AF 14-15 zur (i) Reduzierung der Konzentration von Viren und/oder des afrikanischen Schweinepest-Virus (ASFV) in Tierfutter sowie organischen Einstreumaterial, oder (ii) Inaktivierung des von Viren und/oder afrikanischen Schweinepest-Virus (ASFV) in Tierfutter sowie organischen Einstreumaterial.
- AF17:: Die Verwendung nach AF 1-16, dadurch gekennzeichnet, dass das Tierfutter - Schweinefutter, und/oder - Schweineartigenfutter ist, bevorzugt Schweineartigenfutter für Wildschweine, Hirscheber, Warzenschweine, Buschschweine und/oder Flussschweine.
- AF18:: Die Verwendung nach AF 1-16, dadurch gekennzeichnet, dass das Tierfutter Grünfutter, Melassefutter, Rauhfutter, Stroh, Grobfutter, Saftfutter oder Kraftfutter ist und organische Einstreumaterial aus Stroh, Strohpellets u.a. besteht.
- AF19:: Die Verwendung nach einem oder mehreren der vorherigen AF, dadurch gekennzeichnet, dass das Tierfutter in flüssiger und/oder fester Form verabreicht wird.
- AF20:: Die Verwendung nach einem oder mehreren der vorherigen AF, dadurch gekennzeichnet, dass die Applikation der Kombination oder Zusammensetzung
- bei einer erhöhten ASFV-Last im Tierfutter sowie organischen Einstreumaterial, und/oder
- prophylaktisch, und/oder
- kontinuierlich erfolgt.

## Patentansprüche

1. Verwendung einer Zusammensetzung umfassend
(i) Blütenstände und/oder ein oder mehrere Extrakte weiblicher Blütenstände der Gattung Humulus und/oder
(ii) ein oder mehrere Extrakte von Glycyrrhiza und/oder ein oder mehrere Pflanzenteile von Glycyrrhiza
zur Reduzierung
- von Viren, vorzugsweise des ASF-Virus, in organischer Einstreu; und/oder
- von Viren, vorzugsweise des ASF-Virus, in Futter, vorzugsweise Tierfutter.

2. Die Verwendung nach Anspruch 1, wobei die Gesamtmenge der (i) Blütenstände und/oder Extrakte weiblicher Blütenstände der Gattung Humulus und/oder (ii) Extrakten von Glycyrrhiza und/oder Pflanzenteilen von Glycyrrhiza mindestens 20 Gew.-% der Zusammensetzung umfasst, bezogen auf das Gesamtgewicht der Zusammensetzung.

3. Die Verwendung nach Anspruch 1 oder 2, wobei das Verhältnis, vorzugsweise das Gewichtsverhältnis, der (i) Blütenstände und/oder Extrakte weiblicher Pflanzen der Gattung Humulus und/oder (ii) Extrakten von Glycyrrhiza und/oder Pflanzenteilen von Glycyrrhiza größer 0,5 bis 6,3 ist.

4. Die Verwendung nach einem oder mehreren der vorherigen Ansprüche in einer Dosierung von 0,01Gew.-% bis 0,15 Gew.-%, des Futters, bezogen auf die Gesamtmenge der Trockensubstanz des Futters.

5. Die Verwendung nach einem oder mehreren der vorherigen Ansprüche, wobei die Zusammensetzung zusätzlich
(iii) ein oder mehrere Komponenten unabhängig voneinander ausgewählt aus der Gruppe bestehend aus Traubentrester, Traubenkernextrakt, Traubenkerne, Akazienexsudat, Quillaya saponaria, Seifenrindeextrakt, Weinrebenextrakt, Roselle, Sapindus sapinoria, Kieselgur, Trägerstoff E551 (a-c) und 1,2-Propandiol
enthält.

6. Die Verwendung nach einem oder mehreren der vorherigen Ansprüche, wobei die Zusammensetzung zusätzlich
(iv) ein oder mehrere Komponenten unabhängig voneinander ausgewählt aus der Gruppe bestehend aus Ameisensäure, Essigsäure, Milchsäure, Propionsäure, Buttersäure, Fumarsäure, Äpfelsäure, Weinsäure und Citronensäure enthält; wobei die Säuren teilweise oder vollständig in Form von Ammonium-, Natrium-, Kalium-, Calcium- und/oder Magnesiumsalzen vorliegen können.

7. Die Verwendung nach einem oder mehreren der vorherigen Ansprüche, wobei die Gesamtdosierung der Zusammensetzung bis 1,5 Gew.-%, bezogen auf das Gesamtgewicht der Trockensubstanz des Futters oder organischen Einstreumaterialien erfolgt.

8. Die Verwendung nach einem oder mehreren der vorherigen Ansprüche, wobei die Zusammensetzung die Komponenten (i) und (ii) in einer Menge von 1,5 Gew.-% bis 12 Gew.-%, bezogen auf das Gesamtgewicht der Zusammensetzung, enthält.

9. Die Verwendung nach einem oder mehreren der vorherigen Ansprüche, **dadurch gekennzeichnet, dass** das Tierfutter Schweinefutter, und/oder Schweineartigenfutter ist, bevorzugt Schweineartigenfutter für Wildschweine, Hirscheber, Warzenschweine, Buschschweine und/oder Flussschweine.

10. Die Verwendung nach einem oder mehreren der vorherigen Ansprüche, **dadurch gekennzeichnet, dass** das Tierfutter Grünfutter, Melassefutter, Rauhfutter, Stroh, Grobfutter, Saftfutter oder Kraftfutter ist.

11. Die Verwendung nach einem oder mehreren der vorherigen Ansprüche, **dadurch gekennzeichnet, dass** das Tierfutter in flüssiger und/oder fester Form verabreicht wird.

12. Die Verwendung nach einem oder mehreren der vorherigen Ansprüche, **dadurch gekennzeichnet, dass** die Applikation der Zusammensetzung
- bei einer erhöhten Virenlast,
- vorzugsweise ASFV-Last,
- in Tierfutter und/oder
- in organischen Einstreumaterialien, und/oder
- prophylaktisch, und/oder
- kontinuierlich erfolgt.

13. Die Verwendung nach einem oder mehreren der vorherigen Ansprüche, **dadurch gekennzeichnet, dass** die Zusammensetzung kein Maiskolbenmehl enthält.

14. Zusammensetzung umfassend
(i) Blütenstände und/oder ein oder mehrere Extrakte weiblicher Blütenstände der Gattung Humulus und/oder
(ii) ein oder mehrere Extrakte von Glycyrrhiza und/oder ein oder mehrere Pflanzenteile von Glycyrrhiza und/oder
(iii) zusätzlich eine oder mehrere Komponenten ausgewählt aus der Gruppe bestehend aus Traubentrester, Traubenkernextrakt, Traubenkerne, Akazienexsudat, Quillaya saponaria, Seifenrindeextrakt, Weinrebenextrakt, Roselle, Sapindus sapinoria, Kieselgur, Trägerstoff E551 (a-c) und 1,2-Propandiol und/oder
(iv) ein oder mehrere Komponenten ausgewählt aus der Gruppe bestehend aus Ameisensäure, Essigsäure, Milchsäure, Propionsäure, Buttersäure, Fumarsäure, Äpfelsäure, Weinsäure und/oder Citronensäure, wobei die Säuren teilweise oder vollständig in Form ihrer Ammonium-, Natrium-, Kalium-, Calcium- und/oder Magnesiumsalze vorliegen können;
- vorzugsweise Maiskolbenmehl nicht umfassend;
konfektioniert zur Reduzierung
- von Viren, vorzugsweise des ASF-Virus, in organischer Einstreu; und/oder
- von Viren, vorzugsweise des ASF-Virus, in Futter, vorzugsweise Tierfutter.

15. Die Zusammensetzung gemäß Anspruch 14, definiert wie in einem oder mehreren der Ansprüche 2 bis 13.
